# EUROPÄISCHE PATENTSCHRIFT

(11) **EP 0 643 769 B1**
(45) Veröffentlichungstag und Bekanntmachung des Hinweises auf die Patenterteilung: **30.09.1998**
(21) Anmeldenummer: 94910269.3
(22) Anmeldetag: 31.03.1994
(51) Int. Cl.: C12N 15/29, C07K 14/415, A61K 39/36, G01N 33/53

(54) **REKOMBINANTES LIESCHGRASPOLLENALLERGEN $i(Phl p) II**
RECOMBINANT TIMOTHY GRASS POLLEN ALLERGEN $i(Phl p) II
ALLERGENE RECOMBINE DU POLLEN DE LA PRELE DES PRES $i(Phl p) II

(30) Priorität: 01.04.1993 AT 672/93
(43) Veröffentlichungstag der Anmeldung: 22.03.1995
(73) Patentinhaber: BIOMAY PRODUKTIONS- UND HANDELSGESELLSCHAFT M.B.H., A-4020 Linz (AT)
(72) Erfinder: DOLECEK, Christiane, A-1180 Wien (AT); VRTALA, Susanne, A-1210 Wien (AT); LAFFER, Sylvia, A-1190 Wien (AT); STEINBERGER, Peter, A-1150 Wien (AT); KRAFT, Dietrich, A-1170 Wien (AT); SCHEINER, Otto, A-2380 Perchtoldsdorf (AT); VALENTA, Rudolf, A-2604 Theresienfeld (AT)
(74) Vertreter: Itze, Peter, Dipl.-Ing.
(86) Internationale Anmeldenummer: AT9400039
(87) Internationale Veröffentlichungsnummer: WO9423035

(56) Entgegenhaltungen:
- INTERNATIONAL ARCHIVES OF ALLERGY AND IMMUNOLOGY, Bd.97, 1992, BASEL, CH Seiten 287 - 294 R.VALENTA ET AL. 'Diagnosis of Grass Pollen Allergy with Recombinant Timothy Grass (Phleum pratense) Pollen Allergens' in der Anmeldung erwähnt
- FEBS LETTERS., Bd.335, Nr.3, 13. Dezember 1993, AMSTERDAM NL Seiten 299 - 304 C.DOLECEK ET AL. 'Molecular characterization of Phl p II, a major timothy grass (Phleum pratense) pollen allergen'
- Gentechnologie von A bis Z; Horst Ibelgaufts, VCH Verlagsgesellschaft mbH, Weinheim, Seiten 345-445

## Beschreibung

Gräserpollenallergien gehören zu den wichtigsten pflanzlichen Allergien während des Sommers. Mehr als 20% der Pollenallergiker zeigen allergische Symptome auf Gräserpollen Allergene. Zu den wichtigsten Gräserpollenallergenen gehören Gruppe I (1), Gruppe V (2), Gruppe II/III (3) und Gruppe IV (4, 5) Allergene. Inzwischen wurde auch Profilin als Gräserpollen Allergen entdeckt (7, 8). Die erwähnten Allergene können als immunologisch und strukturell nahe verwandte Moleküle in Pollen unterschiedlicher Grasspezies gefunden werden und verwandte Allergene einer Gruppe zeigen Kreuzreaktivität mit Patienten IgE. Bisher ist eine Reihe dieser Allergene mittels rekombinanter Techniken isoliert und in *E. coli* exprimiert worden (9). Viele von den in *E. coli* exprimierten Allergenen zeigen ähnliche Eigenschaften wie die natürlichen Proteine und können daher für Diagnose und Therapie von allergischen Erkrankungen verwendet werden (10, 11, 12). Es wird nun erstmalig eine molekulare Charakterisierung einer vollständigen cDNA, die für *Phl p* II kodiert, sowie die Expression dieses Proteins in *E. coli* beschrieben. Ein vollständiges rekombinantes Gräserpollenallergen der Gruppe II/III war bisher nicht verfügbar und kann, wie aus den Beispielen ersichtlich ist, wie das natürliche Protein für Verfahren verwendet werden, die auf einer Antigen-Antikörperwechselwirkung beruhen, wie für zelluläre Verfahren Anwendung finden, da sämtliche T-Zell Epitope am rekombinanten Molekül vorhanden sind wie am natürlichen Molekül. Weiters ist das rekombinante *Phl p* II für Verfahren geeignet, die meßbare Mediatorfreisetzung zur Folge haben. Die therapeutische Verwendung des rekombinanten *Phl p* II, basierend auf einer Einwirkung auf immunoregulatorische Prozesse, Antigen-Antikörper-Wechselwirkung, T-Zell Reaktivität und Mediatorfreisetzung folgt aus der strukturellen und biologischen Ähnlichkeit der natürlichen und rekombinanten Proteine. Die vorliegende Erfindung stellt eine vollständige cDNA, die für ein rekombinantes *Phl p* II Allergen kodiert, zur Verfügung. Anhand der von der cDNA abgeleiteten Aminosäuresequenz werden B-Zell und T-Zell Epitope des *Phl p* II Allergens bestimmt. Das rekombinante *Phl p* II Allergen wurde in *E*. *coli* hergestellt und besitzt ähnliche Eigenschaften wie natürliche Gräserpollenallergene der Gruppe II/III. Es folgt daraus, daß das rekombinante *Phl p* II Allergen so wie die natürlichen Allergene der Gruppe II/III für Verfahren verwendet werden kann, die auf einer Antigen-Antikörper-Wechselwirkung, einer antigenabhängigen T-Zellwirkung oder einer antigenabhängigen Mediatorfreisetzung beruhen, wobei aber die rekombinanten Allergene noch den Vorteil der höheren Reinheit und höheren Spezifität aufweisen.

Gemäß Int. Arch.Allergy Immunol.97:287 - 294 (1992) Valenta et al wurde ein Klon zusammmen mit drei anderen Allergenen getestet, der für ein neues Graspollenallergen codieren soll.

Gemäß der nicht vorveröffentlichten Abhandlung in FEBs Letters 335:299 - 304 (1993) Dolecek et al, stellte sich heraus, daß es sich bei diesem Klon um Phl p II handelt, wobei erst in dieser nachveröffentlichten Abhandlung die Einzelheiten und Wirkungen von Phl p II beschrieben sind.

### Material und Methoden:

### 1. Konstruktion der cDNA Genbank

Lieschgraspollen (Allergon AB Engelholm, Schweden), der mit Hilfe von Licht- und Elektronenmikroskopie auf Reinheit untersucht worden war, wurde zur Isolierung von polyadenylierter RNA verwendet (13). cDNA Synthese wurde mit oligo-dT und random Primern durchgeführt, die Enden der cDNA wurden mit T4-Polymerase glattverdaut und mit EcoRI-Linkern versehen. Die cDNA mit Linkern wurde in dephosphorylierte Lambda gtl1 Arme ligiert und verpackt. Es ergab sich eine cDNA Genbank von 800.000 unabhängigen Klonen (13).

### 2. Screening der cDNA Genbank. Subklonierung und DNA Sequenzanalyse

IgE Screening der Lieschgrasspollen cDNA Genbank wurde durchgeführt wie von Breiteneder et al., beschrieben (14). IgE bindende Klone wurden angereichert und aus diesen Klonen wurde Phagen-DNA präpariert (15). Mittels Kpn I und Sac I Schnitten konnten zwei DNA Fragmente erhalten werden, die beide Teile der vollständigen *Phl p* II cDNA und flankierende lambda gtl1 Sequenzen enthielten. Die entstandenen KpnI/SacI und SacI DNA Fragmente wurden in das Plasmid pUC 18 subkloniert und *E. coli* XL-1 Blue damit tranformiert. Durch Restriktionsanalyse wurden geeignete Klone identifiziert und mittels lambda gtl1 forward sequencing primer (22-mer) und lambda gtll reverse sequencing primer (22-mer), Clontech Laboratories, Palo Alto, USA sowie mittels M13 pUC18 forward and reversed primern, Boehringer-Mannheim, Deutschland, nach Sanger (16) beidsträngig sequenziert.

### 3. RNA (Northern) Blots

10 µg von Gesamt-RNA aus Pollen von Lieschgras *(Phleum pratense)* und Lolchgras *(Lolium perenne)* wurden mit Hilfe einer denaturierenden Gelelektrophorese aufgetrennt und auf Nitrocellulose geblottet (17). Zur Isolierung der cDNA die für *Phl p* II kodiert wurde ausgehend von rekombinanten Phagen das entsprechende DNA Insert mittels PCR amplifiziert. Es wurden jeweils 5 picomol primer (lambda gtll forward sequencing primer (22-mer) und lambda gtll reverse sequencing primer (22-mer), Clontech Laboratories,USA) eingesetzt. Das PCR-Produkt wurde auf ein 1%-iges Agarosegel aufgetragen und die Bande wurde mittels DEAE-Ionenaustauscherpapier eluiert (18). Die gewonnene DNA wurde mittels random priming ³²P-markiert (19). Prähybridisierung und Hybridisierung wurden nach Standardmethoden durchgeführt (15). Die Blots wurden mit 3.0xSSC (20xSSC = 3M NaCl, 0,3M Na-Citrat, pH 7,0), 0.1%SDS (Natriumdodecylsulfat); 1.5 SSC, 0.1%SDS und anschließend mit 0.75% SSC, 0.1% SDS bei 50°C gewaschen und autoradiographiert (Hyperfilm MP, Amersham, London, UK).

### 4. Expression der Phl p II cDNA in lysogenen E. coli Y1089 als β-Galaktosidasefusionsprotein

Mit Hilfe von IgE Screening wurde ein vollständiger cDNA Klon erhalten, der für ein *Phl p* II Allergen kodiert. Mit rekombinanten Lambda gtl1 Phagen wurde der lysogene *E. coli* Stamm Y1089 infiziert und aus dem Ansatz wurde das β-Galactosidase-Fusionsprotein gewonnen (20). Der Ansatz wurde in einem 7.5% Polyacrylamidgel elektrophoretisch aufgetrennt (21) und auf Nitrocellulose geblottet (22). Das Fusionsprotein wurde mit Hilfe von Serum-IgE von graspollenallergischen Patienten und einem jodmarkierten Kaninchen-anti-human IgE Antikörper (Pharmacia, Uppsala, Schweden) detektiert.

Die angeschlossenen Figuren dienen der Illustration der Verwendbarkeit des rekombinanten *Phl p* II Allergens für Verfahren, die auf Antigen-Antikörper-Wechselwirkung, antigenabhängiger Mediatorfreisetzung, sowie antigenabhängiger zellulärer Reaktivität beruhen. Figur 1 zeigt die cDNA und die davon abgeleitete Aminosäuresequenz des rekombinanten *Phl p* II Allergens. Es ist die vollständige cDNA Sequenz des *Phl p* II Allergens angeführt. Eine 24 Basen lange nichtkodierende Sequenz wurde am 5' Ende der cDNA vorgefunden, worauf eine 78 Basen lange Führungssequenz folgt, die für das in der Abbildung unterstrichene Signalpeptid kodiert (23). Die Aminosäuresequenz des reifen Proteins kann ab Base 78 abgeleitet werden und beginnt mit Valin. Das Stopcodon TAG, welches die kodierende Sequenz am 3' Ende abbricht, ist mit einem Stern gekennzeichnet. Die 3' nicht kodierende Sequenz wird im poly A Schwanz beendet.

Figur 2 veranschaulicht die Ähnlichkeit der Aminosäuresequenz des rekombinanten *Phl p* II Allergens mit anderen Gruppe II/III Gräserpollenallergenen.

Figur 3 gibt die Beschreibung von B-Zell Epitopen des rekombinanten *Phl p* II Allergens wieder, und Figur 4 jene der T-Zell Epitope.

Figur 5 zeigt die Reaktivität des rekombinanten *Phl p* II Allergens mit Serum IgE von Graspollenallergikern. Rekombinantes *Phl p* II wurde als β-Galaktosidase Fusionsprotein in lysogenen *E. coli* Y1089 mittels Phageninfektion und Induktion mit IPTG exprimiert. Die *E. coli* Proteine, welche das rekombinante *Phl p* II enthalten, wurden elektrophoretisch aufgetrennt und auf Nitrozellulose übertragen. In Spur 1 wurde Serum IgE von einem Gruppe II/III reaktiven Patienten verwendet, in den Spuren 2 und 3 wurde mit IgE von Allergikern detektiert, die keine Reaktion mit Gruppe II/III Allergenen zeigen, und Spur 4 zeigt die Kontrolle mit Serum einer nicht allergischen Kontrollperson.

Figur 6 gibt eine Tabelle wieder, die den Prozentsatz von graspollenallergischen Patienten mit IgE-Reaktivität gegen bestimmte Graspollenallergene auflistet. Es wird die Häufigkeit der Reaktivität von Graspollenallergikern mit verschiedenen Gräserpollenallergenen gezeigt. Die Werte sind Richtwerte, die durch Testen mit natürlichen und rekombinanten Gräserpollenallergenen in einer repräsentativen Anzahl von Patienten erhoben wurden.

Figur 7 belegt, daß rekombinantes *Phl p* II gleiche IgE-Epitope wie natürliche Gruppe II/III Allergene-IgE-Inhibition trägt. Das Serum eines Graspollenallergikers, der mit Gruppe II/III Allergenen (10-12kD) und Gruppe I Allergenen (etwa bei 30 kD) IgE-Reaktivität zeigt, wurde mit rekombinantem *Phl p* II (Spur 2), rekombinantem *Phl p* I (Spur 3), rekombinantem *Bet v* I (Spur 4), oder E. coli-Proteinen (Spur 1) vorinkubiert. Vorinkubation mit rekombinanten *Phl p* II bringt die IgE-Bindung an natürliches *Phl p* II im 12 kD Bereich fast völlig zum verschwinden, während die Reaktivität mit *Phl p* I bei 30 kD nicht beeinträchtigt wird. Rekombinantes *Phl p* I reduziert nur die Bindung an natürliches *Phl p* I, hat aber keine Wirkung auf die IgE-Bindung an *P h l p* II. Die Vorinkubation mit Kontrollproteinen, rekombinanten *Ber v I* und *E. coli* Proteinen beeinträchtigt die IgE-Bindung an natürliches *Phl p* I und *Phl p* II nicht. Daraus folgt, daß rekombinantes *Phl p* II ähnliche oder gleiche IgE-Epitope wie natürliches *Phl p* II trägt, jedoch keine antigene Verwandtschaft mit natürlichen oder rekombinanten Allergenen der Gruppe I gegeben ist.

Figur 8 verdeutlicht die Hybridisierung der cDNA, die für *Phl p* II kodiert, mit mRNA aus Lieschgras und Lolchgras. Gesamt RNA wurde aus Lolchgras (Spurl) und Lieschgras (Spur 2) Pollen isoliert, 10,µg im denaturierenden Agarosegel aufgetrennt und auf Nitrozellulose geblottet. Die mit Phosphor 32 markierte cDNA, für kodierend für *Phl p* II, hybridisiert sowohl mit Lolchgras als auch Lieschgras RNA etwa in Höhe der 18S RNA aber auch deutlich darunter bei etwa 600 Basen Transkriptgröße. Die Kreuzhybridisierung zeigt die strukturelle Ähnlichkeit der Transkripte welche für Gruppe II/III Allergene in verschiedenen Grasspezies kodieren.

Figur 9 A und B zeigt die Reaktivität des rekombinanten *Phl p* II Allergens mit einem Antikörper spezifisch für Gruppe II/III Gräserpollenallergene

A: Lambda gtl1 Phagen, die Lieschgraspollenallergene und das Hauptallergen der Birke, *Bei v* I (Co), exprimieren sowie nicht rekombinante Phagen (λ) wurden im Dot Blot Verfahren mit Antikörpern spezifisch für Graspollenallergene getestet. 4B 1 bindet an Gruppe V Allergene, R4 detektiert Gruppe I Allergene, und R5 identifiziert Gruppe V und Gruppe II/III Allergene. B: Die Graphik illustriert die Klonbezeichnung. Rekombinantes *Phl p* II wird von Klon A exprimiert.

### Beispiele:

### Sequenzanalyse des rekombinanten Phl p II Allergens-Ähnlichkeit mit anderen Gruppe II/III Allergenen

Die DNA Sequenz des *Phl p* II Allergens wurde durch Sequenzierung der cDNA nach der Methode von Sanger (16) bestimmt. Abbildung 1 zeigt die bestimmte DNA-Sequenz und die daraus abgeleitetete Aminosäuresequenz. Ein dem reifen Protein voranstehendes Signalpeptid, das signifikante Homologie mit anderen eukaryotischen Signalpeptiden zeigt, beweist eindeutig, daß Gruppe II/III Allergene von distinkten Genabschnitten kodiert werden und nicht durch proteolytischen Zerfall aus Gruppe I Allergenen entstehen. Die in Abbildung 2 gezeigte hohe Sequenzhomologie (ungefähr 70% Sequenidentität) des rekombinanten *Phl p* II mit den homologen Proteinen aus dem Lolchgras *(Lol* p II und *Lol p* III) zeigt die enge strukturelle Verwandtschaft dieser Proteine und liefert damit die molekulare Basis für die immunologische Verwandtschaft von Gruppe II/III Allergenen verschiedener Spezies.

### Bestimmung der B-Zell und T-Zell Epitope des rekombinanten Phl p II Allergens

Anhand der abgeleiteten Aminosäuresequenz des *Phl p* II Allergens konnten die B-Zell und T-Zell Epitope unter Verwendung geeigneter Computerprogramme (24, 25) bestimmt werden. Die relevanten B-Zell und T-Zell Epitope sind in den Figuren 3 und 4 zusammengefaßt. Synthetische Peptide, die B-Zell Epitopen entsprechen, binden IgE von Graspollenallergikern, während synthetische Peptide, die T-Zell Epitopen entsprechen, T-Zellen von Graspollenallergikern zur Proliferation anregen und erhöhte H3-Thymidinaufnahme zur Folge haben.

### Expression der cDNA kodierend für Phl p II in E. coli als rekombinantes Phl p II Allergen

Rekombinante Phagen, die die cDNA für *Phl p* II enthalten. wurden zur Infektion mit lysogenem *E. coli* Y 1089 verwendet. Rekombinantes β-Galaktosidase Fusionsprotein wurde durch Induktion mit IPTG (Isopropyl-β-Thiogalaktosid) in Flüssigkultur gewonnen (20). Das Kontrollprotein β-Galaktosidase zeigte keine IgE-Bindung am Western Blot während das rekombinante *Phl p* II Fusionsprotein spezifisch mit Serum IgE von Patienten, die mit Gruppe II/III Allergenen verschiedener Grasspezies reagierten, deutliche IgE-Bindung zeigte.

### IgE-Bindungsfähigkeit des rekombinanten Phl p II Allergens

Serum eines Graspollenallergikers, der mit natürlichen Allergenen der Gruppe II/III reagiert, wurde mit rekombinanten *Phl p* II vorinkubiert und damit Gruppe II/III spezifisches IgE abgebunden. Figur 6 zeigt, daß die Bindung an natüliche Gruppe II/III Allergene fast vollständig durch die Vorinkubation ausgelöscht wird, während die Bindung an Gruppe I und Gruppe V Allergene nicht beeinflußt wurde. Dies zeigt, daß die IgE-Epitope von natürlichen Gruppe II/III Allergenen durch rekombinantes *Phl p* II abgedeckt werden, und daß kaum relevante Kreuzreaktivität zwischen Gruppe I, Gruppe V und Gruppe II/III Allergenen bestehen.

### Kreuzhybridisierung der Phl p II cDNA mit Lol p II/III mRNA

Gesamt RNA von Lolchgras und Lieschgraspollen wurde isoliert, im denaturienden Agarosegel aufgetrennt und auf Nitrocellulosemembranen übertragen. Die Membran wurde mit einer vollständigen cDNA, die für *Phl p* II kodiert, hybridisiert. Hybridisierende Banden finden sich jeweils in Höhe der 18S ribosomalen RNA sowie deutlich darunter. Die Hybridisierung ist deutlich intensiver mit Lieschgras RNA als mit Lolchgras, RNA obwohl nach der Gelfärbung mit Ethidiumbromid etwa gleiche Mengen Gesamt RNA verwendet wurden. Dennoch konnte unter stringenten Bedingungen Kreuzhybridisierung erzielt werden. Das größere Transkript stellt unter Umständen eine größere und noch unreife RNA dar, da die Hybridisierung auch stringentem Waschen standhielt. Dieses Beispiel soll die Homologie der Gruppe II/III Gräserpollenallergene verschiedener Spezies dokumentieren.

### Literaturnachweis:

1.Freidhoff, L.R., Ehrlich-Kautzky, E.,Grant, J.H., Meyers, D.A., and Marsh, D.G. (1986) J Allergy Clin Immunol 78, 1190-1201
2. Matthiesen, F., Lowenstein, H. Clin Exp Allergy 21, 309-320.
3. Ansari, A.A.,Shenbagamurthi, P.,and Marsh, D.G. (1989) J. Biol. Chem. 264, 11181-11185
4. Kisil, F. T., Jaggi, K. S., Lin, Z. W., Ekramodullah, A. K. M. (1989) in Sehon A. H., Kraft, D., Kunkel, G. eds. Epitopes of Atopic Allergens, 22-25.
5. Jaggi, K. S., Ekramodullah, A. K. M., Kisil, F. T., Dzuba-Fischer, J. M. M., Rector, E. S., and Sehon, A. H. (1989) J Allergy Clin Immunol 83, 845-852.
6. van Ree, R., Driessen, N. B. M., van Leeuwen, W. A., Stapel, S. O., and Aalberse, R. C. (1992) Clin Exp Allergy.
7. Valenta, R., Duchêne, M., Ebner, C., Valent, P., Sillaber, C., Deviller, P., Ferreira, F., Tejkl, M., Edelmann, H., Kraft, D., and Scheiner, O. (1992) J Exp Med 175, 377-385.
8. Valenta, R., Duchêne, M., Vrtala, S., Valent, P., Sillaber, C., Ferreira, F., Tejkl, M., Hirschwehr, R., Ebner, C., Kraft, D., and Scheiner, O. (1993) Int Arch Allergy Appl Immunol 99, 271-273.
9. Scheiner, O., Bohle, B., Breitenbach, M., Breiteneder, H., Duchêne, M., Ebner, C., Ferreira, F., Hirschwehr, R., Hoffmann-Sommergruber, K., Pettenburger, K., Rumpold, H., Steiner, R., Tejkl, M., Valenta, R., and Kraft, D. (1992) in: Advances in Allergology and Clinical Immunology, Godard, P., Bousquet, J., and Michel, F. B. eds. The Parthenon Publishing Group.
10. Valenta, R., Duchêne, M., Vrtala, S., Birkner, T., Ebner, C., Hirschwehr, R., Breitenbach, M., Rumpold, H., Scheiner, O., and Kraft, D. (1991) J Allergy Clin Immunol 88, 889-894.
11. Valenta, R., Vrtala, S., Ebner, C., Kraft, D., and Scheiner, O. (1992) Int Arch Allergy Appl Immunol 97, 287-294
12. Valenta, R., Sperr, W. R., Ferreira, F., Valent, P., Sillaber, C., Tejkl, M., Duchêne, M., Ebner, C., Lechner, K., Kraft, D., and Scheiner, O. (1993). J Allergy Clin Immunol 91, 88-97.
13. Vrtala, S., Sperr, W. R., Reimitzer, I., vanRee, R., Laffer, S., Müller, W.-D., Valent, P., Lechner, K., Rumpold, H., Kraft, D., Scheiner, O., and Valenta, R. (1993) J Immunol (accepted provided revision).
14. Breiteneder, H., Pettenburger, K., Bito, A., Valenta, R., Kraft, D., Rumpold, H., Scheiner, O., and Breitenbach, M. (1989) EMBO J 8, 1935-1938
15. Ausubel, F. M. (1990) in Current protocols in molecular biology, Wiley, New York.
16. Sanger, F., Nicklen, S., Coulson, A. R. (1977) Proc Natl Acad Sci USA 74, 5463-5468.
17. Valenta, R., Breiteneder, H., Pettenburger, K., Breitenbach, M., Rumpold, H., Kraft, D., and Scheiner, O. (1991) J Allergy Clin Immunol 87, 677-682.
18. Sambrook, J., Fritsch, E. F., Maniatis, T. (1989) Molecular cloning: a laboratory manual. Cold Spring Harbor Laboratory Press.
19. Feinberg, A. P., and Vogelstein, B. (1983) Anal Biochem 132, 6-13.
20. Huynh, T. V., Young, R. A., Davis, R. W. (1985) in: cDNA cloning, Oxford, IRL Press, vol I, 49-78.
21. Laemmli U. K. (1970) Nature 227, 680-685.
22. Towbin, H., Staehelin, T., Gordon, J. (1979) Proc Natl Acad Sci USA 76, 4350-4354.
23. Gavel, Y., and Heijne, G. (1990) FEBS Lett 261, 455-458.
24. Kyte, J. and Doolittle, R. F. (1982) J Mol Biol 157, 105-132.
25. Margalit, H., Spouge, J. L., Cornette, J. L., Cease, K. B., Delisi, C., and Berzofsky, J. A. (1987) J Immunol 138, 2213-2229.

## Patentansprüche

1. Rekombinantes DNA Molekül, dadurch gekennzeichnet, daß es eine Nukleinsäuresequenz aufweist, die für ein Polypeptid kodiert, das die Antigenität des Allergens *Phl p* II (Lieschgras Pollen Allergen), insbesondere monokotyledoner Gewächse, besitzt oder für ein Peptid, das mindestens ein Epitop dieses Allergens aufweist, sowie eine Nukleinsäuresequenz, die mit der genannten Nukleinsäuresequenz unter stringenten Bedingungen hybridisiert, wobei die Nukleinsäuresequenz mit der in Fig. 1 dargestellten gesamten Sequenz oder Teilbereichen derselben in homologer Weise übereinstimmt, bzw. durch Degeneration aus der in Fig. 1 dargestellten Sequenz ableitbar ist.

2. Rekombinantes DNA Molekül nach Anspruch 1, dadurch gekennzeichnet, daß es funktionell mit einer Expressionskontrollsequenz zu einem Expressionskonstrukt verbunden ist.

3. Wirtssystem, dadurch gekennzeichnet, daß es mit einem rekombinanten Expressionskonstrukt nach Patentanspruch 2 transformiert ist.

4. Aus einem DNA-Molekül nach Anspruch 1 abgeleitetes rekombinantes oder synthetisches Protein oder Polypeptid, dadurch gekennzeichnet, daß es eine Aminosäuresequenz aufweist, die der in Fig. 1 gezeigten Sequenz entspricht.

5. Rekombinantes oder synthetisches Protein oder Polypeptid nach Patentanspruch 4, dadurch gekennzeichnet, daß es ein Fusionsprodukt darstellt, das die Antigenität des *Phl p* II Allergens des Lieschgrases aufweist und einen zusätzlichen Polypeptidanteil aufweist, wobei das gesamte Fusionsprodukt von der DNA eines Expressionskonstrukts gemäß Anspruch 2 kodiert wird.

6. Rekombinantes oder synthetisches Protein oder Polypeptid nach Patentanspruch 5, dadurch gekennzeichnet, daß der besagte zusätzliche Polypeptidanteil β-Galactosidase oder ein anderes zur Fusion geeignetes Polypeptid ist.

7. Diagnostisches oder therapeutisches Reagens, dadurch gekennzeichnet, daß es ein synthetisches Protein oder Polypeptid gemäß einem der Patentansprüche 4 bis 6 enthält.

8. Verfahren, zum *in vitro -* Nachweis der Allergie eines Patienten gegen das *Phl p* II Allergen, dadurch gekennzeichnet, daß die Reaktion der IgE Antikörper im Serum des Patienten mit einem rekombinanten oder synthetischen Protein oder Polypeptid nach einem der Patentansprüche 4 bis 6 gemessen wird.

9. Verfahren, zum *in vitro -* Nachweis der zellulären Reaktion auf das *Phl p* II Allergen, dadurch gekennzeichnet, daß ein rekombinantes oder synthetisches Protein oder Polypeptid nach einem der Patentansprüche 4 bis 6 zur Stimulierung oder Hemmung der zellulären Reaktion eingesetzt wird.

## Claims

1. A recombinant DNA molecule, characterized in that it has a nucleic acid sequence that codes for a polypeptide which has the antigenicity of the *Phl p* II allergen (Timothy Grass pollen allergen), especially monocotyledonous plants, or for a peptide having at least one epitope of this allergen, and a nucleic acid sequence which hybridizes with said nucleic acid sequence under stringent conditions, where the nucleic acid sequence is homologous to the total sequence shown in Figure 1 or subregions thereof, or it can be derived by degeneration of the sequence shown in Figure 1.

2. A recombinant DNA molecule according to Claim 1, characterized in that it is functionally connected to an expression control sequence to form an expression construct.

3. A host system, characterized in that it is transformed with a recombinant expression construct according to claim 2.

4. A recombinant or synthetic protein or polypeptide derived from a DNA molecule according to Claim 1, characterized in that it has an amino acid sequence corresponding to that shown in Figure 1.

5. A recombinant or synthetic protein or polypeptide according to claim 4, characterized in that it is a fusion product having the antigenicity of the *Phl p* II allergen from Timothy Grass and an additional polypeptide component, where the entire fusion product is coded by the DNA of an expression product according to Claim 2.

6. A recombinant or synthetic protein or polypeptide according to claim 5, characterized in that said additional polypeptide is β-galactosidase or another polypeptide suitable for fusion.

7. A diagnostic or therapeutic reagent, characterized in that it contains a synthetic protein or polypeptide according to one of the claims 4 through 6.

8. A method of *in vitro* detection of a patient's allergy to the *Phl p* II allergen, characterized in that the reaction of the IgE antibodies in the patient's serum is measured with a recombinant or synthetic protein or polypeptide according to one of the claims 4 through 6.

9. A method of *in vitro* detection of the cellular reaction to the *Phl p* II allergen, characterized in that a recombinant or synthetic protein or polypeptide according to one of the claims 4 through 6 is used to simulate or inhibit the cellular reaction.

## Revendications

1. Molécule D'A.D.N. recombinant caractérisée en ce qu'elle présente une séquence d'acide nucléique codant pour un polypeptide qui possède les caractéristiques antigéniques de l'allergène *PhL p* II (allergène du pollen de la laîche), notamment des monocotylédones, ou pour un peptide présentant au moins un épitope de cet allergène, ainsi qu'une séquence d'acide nucléique laquelle hybride avec la séquence d'acide nucléique mentionnée dans des conditions rigoureuses, la séquence d'acide nucléique concordant de manière homologue avec l'intégralité ou des fragments de la séquence représentée à la fig. 1 ou pouvant découler par dégénération de cette même séquence.

2. Molécule D'A.D.N. recombinant selon la revendication 1 caractérisée en ce qu'elle est liée fonctionnellement à une matrice d'expression au moyen d'une séquence de contrôle d'expression.

3. Système-hôte caractérisé en ce qu'il est transformé avec une matrice d'expression recombinante selon la revendication 2.

4. Protéine ou polypeptide recombinant ou synthétique découlant d'une molécule d'A.D.N. selon la revendication 1 caractérisé en ce qu'elle ou il présente une séquence d'acides aminés correspondant à la séquence représentée sur la figure 1.

5. Protéine ou polypeptide recombinant ou synthétique selon la revendication 4 caractérisé en ce qu'elle ou il représente un produit de fusion présentant les caractéristiques antigéniques de l'allergène *Phl p* II de la laîche et présentant un fragment de polypeptide supplémentaire, l'intégralité du produit de fusion étant codée par l'A.D.N, d'une matrice d'expression selon la revendication 2.

6. Protéine ou polypeptide recombinant ou synthétique selon la revendication 5 caractérisé en ce que ledit fragment de polypeptide supplémentaire est du β-galactosidase ou un autre polypeptide approprié à la fusion.

7. Réactif à des fins de diagnostic ou thérapeutiques caractérisé en ce qu'il contient une protéine ou un polypeptide synthétique selon l'une des revendications 4 à 6.

8. Procédé permettant de montrer *in vitro* qu'un patient est allergique à l'allergène *Phl p* II caractérisé en ce qu'on mesure la réaction des anticorps IgE dans le sérum sanguin du patient à l'aide d'une protéine ou d'un polypeptide recombinant ou synthétique selon l'une des revendications 4 à 6.

9. Procédé permettant de montrer *in vitro* la réaction cellulaire à l'allergène *Phl p* II caractérisé en ce qu'on utilise une protéine ou un polypeptide recombinant ou synthétique selon l'une des revendications 4 à 6 pour stimuler ou inhiber la réaction cellulaire.
